# EUROPÄISCHE PATENTSCHRIFT

(11) **EP 1 893 111 B1**
(45) Veröffentlichungstag und Bekanntmachung des Hinweises auf die Patenterteilung: **26.03.2014**
(21) Anmeldenummer: 06762141.7
(22) Anmeldetag: 22.06.2006
(51) Int. Cl.: A61B 17/88, B21D 7/06

(54) **CHIRURGISCHE BIEGEZANGE UND BIEGEZANGENSYSTEM**
SURGICAL BENDING FORCEPS AND BENDING FORCEPS SYSTEM
PINCE A CINTRER CHIRURGICALE ET SYSTEME DE PINCE A CINTRER

(30) Priorität: 23.06.2005 DE 102005029165
(43) Veröffentlichungstag der Anmeldung: 05.03.2008
(73) Patentinhaber: Stryker Leibinger GmbH & Co. KG, 79111 Freiburg (DE)
(72) Erfinder: SCHMUCK, Manfred, 78570 Mühlheim-Stetten (DE); GREINER, Karl, 78570 Mühlheim (DE); KLEIN, Ludger, 79102 Freiburg (DE)
(74) Vertreter: Röthinger, Rainer
(86) Internationale Anmeldenummer: PCT/EP2006/006027
(87) Internationale Veröffentlichungsnummer: WO 2006/136418

(56) Entgegenhaltungen:
- DE-A1- 10 301 692
- DE-B- 1 087 990
- DE-C- 172 419
- US-A- 4 488 425
- US-A- 5 490 409

## Beschreibung

### Gebiet der Erfindung

Die Erfindung betrifft eine Biegezange für chirurgische Elemente, wie sie beispielsweise zum Biegen von Knochenplatten für den craniomaxillofacialen Bereich verwendet wird. Die Erfindung betrifft ferner ein die Biegezange umfassendes Biegezangensystem.

### Hintergrund der Erfindung

Im Vorfeld oder während eines chirurgischen Eingriffs ist es oftmals erforderlich, Implantate und andere chirurgische Elemente an anatomische Gegebenheiten anzupassen. Die anatomischen Gegebenheiten können beispielsweise durch die Krümmung eines Knochens oder den Verlauf einer Fraktur bestimmt sein. Zum Anpassen von chirurgischen Elementen an anatomische Gegebenheiten steht dem Chirurgen ein breites Instrumentarium zur Verfügung. Zu diesem Instrumentarium zählen beispielsweise Schneidzangen, Biegezangen, Schränkeisen und ähnliche Hilfsmittel.

Aus der US 4,474,046 ist eine Dreipunkt-Biegezange für chirurgische Stäbe bekannt. Die Biegezange besitzt zwei um eine gemeinsame Drehachse schwenkbare Branchen. Im Bereich der gemeinsamen Drehachse ist ein Biegestempel vorgesehen. Dem Biegestempel sind zwei Gegenlager zugeordnet, die an freien Enden der Branchen ausgebildet sind. Bei einer Betätigung der Branchen bleibt der Biegestempel ortsfest und die beiden Gegenlager führen Bewegungen auf elliptischen Bahnen um den Biegestempel herum aus. Als Folge dieser Bewegungen wird eine zwischen dem Biegestempel und den Gegenlagern angeordnete Stange um den Biegestempel herum gebogen. Um Stangen unterschiedlichen Durchmessers biegen zu können (oder zum Erzielen unterschiedlicher Biegeradien), besitzt der Biegestempel eine abschnittsweise unterschiedliche Außenkontur. Durch Drehen des Biegestempels um die Drehachse der Branchen lässt sich ein gewünschter Konturabschnitt auswählen, mittels dessen der Biegestempel mit der zu biegenden Stange zusammenwirken soll.

Die US 5,490,409 offenbart eine weitere Dreipunkt-Biegezange mit einem drehbaren, exzentrisch gelagerten Biegestempel. Um den drehbaren Biegestempel in einer gewünschten Winkelstellung sicher arretieren zu können, ist ein Mechanismus mit zwei über ein Gelenk miteinander gekoppelten Armen vorgesehen. Im Bereich des Gelenks ist ein Führungsstift angeordnet, der bei einer Betätigung der Biegezange eine lineare Bewegung ausführt. Dabei gleitet der Führungsstift innerhalb eines auf der Rückseite des Biegestempels ausgebildeten Kanals, um den Biegestempel winkelfest zu fixieren.

Aus der US 5,651,283 ist eine multifunktionale Biegezange für lineare Knochenplatten bekannt. Mittels der Biegezange kann eine lineare Knochenplatte sowohl in einer Vorzugsebene der Knochenplatte als auch aus der Vorzugsebene heraus gebogen werden. Das Biegen der Knochenplatte in der Vorzugsebene erfolgt mittels einer Dreipunkt-Mechanik. Die Dreipunkt-Mechanik umfasst zwei im Kopfbereich einer ersten Branche vorgesehene Gegenlager sowie einen Biegestempel, der an einem im Kopfbereich der ersten Branche schwenkbar gelagerten Maulabschnitt ausgebildet ist. Der Maulabschnitt ist über ein Gelenk mit einer zweiten Branche gekoppelt. Ein Verbindungssteg zwischen der ersten und der zweiten Branche ist mit seinem ersten Ende innerhalb eines in der ersten Branche ausgebildeten Schlitzes geführt und mit seinem zweiten Ende an der zweiten Branche angelenkt.

Es hat sich herausgestellt, dass der aus der US 5,651,283 bekannte Ansatz, den Biegestempel an einem schwenkbaren Maulabschnitt vorzusehen, nachteilhaft ist. Ein Nachteil besteht beispielsweise darin, dass der Biegestempel nur begrenzt auslenkbar ist. Aufgrund dieser begrenzten Auslenkbarkeit ist der Anwendungsbereich der Biegezange im Wesentlichen auf Knochenplatten mit eng begrenzten geometrischen Dimensionen bestimmt.

Aus der DE 103 01 692 A1 ist eine weitere Dreipunkt-Biegezange bekannt. Die Biegezange umfasst einen Biegestempel, der bezüglich zweier Gegenlager verschwenkbar ist. Im Gegensatz zu der Biegezange der US 5,651,283 ist zwar eine ausreichende Auslenkbarkeit des Biegestempels gegeben, so dass Knochenplatten mit unterschiedlichen geometrischen Dimensionen gebogen werden können. Allerdings kann sich während eines Biegevorgangs die Schwenkbewegung des Biegestempels destabilisierend auf die Lage der zu biegenden Knochenplatte auswirken.

Ferner ist aus der DE 1 087 990 B eine Zange bekannt, die sowohl zum Ausstanzen als auch zum Biegen von Vorhangschienen dient. Die Zange besitzt an ihren Backenenden Stanzwerkzeuge, mit deren Hilfe dreieckförmige Ausschnitte ausgestanzt werden. Des Weiteren verfügt sie über eine Widerlagerrolle, die mittels eines mit den beiden Zangengriffen verbundenen Kniehebels betätigt werden kann. Als Gegenhalter zu der Widerlagerrolle dienen zwei Auflagernocken, die unterhalb des Stanzwerkzeuges an den Schultern der Griffschenkel der Zange angebracht sind. Die Widerlagerrolle und die Auflagernocken haben jeweils an ihrer Wurzel einen geringeren Querschnitt als an ihrem freien Ende. Dadurch entsteht eine spezielle Profilierung zur Aufnahme der T-förmigen Vorhangsschiene.

Der Erfindung liegt die Aufgabe zugrunde, eine Biegezange für chirurgische Elemente wie Knochenplatten anzugeben, die einen breiten Anwendungsbereich besitzt und ein präzises Biegen gestattet. Eine weitere Aufgabe der vorliegenden Erfindung besteht darin, ein die Biegezange umfassendes Biegezangensystem bereitzustellen.

### Kurzer Abriss der Erfindung

Eine erfindungsgemäße Biegezange für chirurgische Elemente umfasst zwei relativ zueinander schwenkbare Branchen, die mit zusammen wenigstens zwei ersten Gegenlagern für das zu biegende chirurgische Element (z.B. starr oder gelenkig) gekoppelt sind, einem ersten Biegestempel zum Zusammenwirken mit dem chirurgischen Element in einem Bereich zwischen den beiden ersten Gegenlagern und einer Betätigungseinrichtung für den ersten Biegestempel, welche eine Betätigungsbewegung der Branchen in eine Linearbewegung des ersten Biegestempels in Richtung auf das chirurgische Element umsetzt, wobei sich die ersten Gegenlager während der Betätigungsbewegung der Branchen auf jeweils einer elliptischen Bahn auseinander bewegen.

Die Betätigungseinrichtung für den ersten Biegestempel kann derart ausgebildet sein, dass der erste Biegestempel von der Betätigungseinrichtung entlang einer (gedachten) Geraden bewegbar ist. Optional können Führungsmittel vorgesehen werden, welche die Linearbewegung des ersten Biegestempels stabilisieren.

Eine mögliche Ausbildung der Betätigungseinrichtung umfasst ein Getriebe zum Umsetzen der Schwenkbewegung der Branchen in die gewünschte Linearbewegung des Biegestempels. Das Getriebe kann eine Übersetzung derart aufweisen, dass die Schwenkbewegung der Branchen in einem vergleichsweise weiten axialen Versatz Ellipsenlenker-Getriebes ausgebildet sein. Beispielhafte Ellipsenlenker-Getriebe sind in den Kapiteln 3.4.5.7.1. und 3.4.5.7.2. des Lehrbuchs von S. Hildebrand, Feinmechanische Bauelemente, Karl Hanser Verlag, München beschrieben. Die beschriebenen Ellipsenlenker-Getriebe, und Abwandlungen hiervon, sind geeignet, um eine Betätigungsbewegung der Branchen in eine lineare Biegestempelbewegung umzusetzen. Hierzu kann das Getriebe mit jeder der beiden Branchen sowie mit dem Biegestempel gelenkig gekoppelt sein.

Bei einer Ausführungsform der Biegezange umfasst die Betätigungseinrichtung (also beispielsweise das Ellipsenlenker-Getriebe) wenigstens einen ersten Hebel, der mit einer ersten der beiden Branchen und mit dem ersten Biegestempel gelenkig gekoppelt ist. Die Betätigungseinrichtung kann ferner einen zweiten Hebel umfassen, der mit der zweiten Branche und ebenfalls mit dem ersten Biegestempel gelenkig gekoppelt wird. Mittels eines gemeinsamen Gelenks können der erste und der zweite Hebel miteinander sowie mit dem ersten Biegestempel gekoppelt sein. Das gemeinsame Gelenk ist gemäß einer ersten Variante unmittelbar im Bereich des ersten Biegestempels ausgebildet. Gemäß einer zweiten Variante ist der erste Biegestempel beabstandet von dem gemeinsamen Gelenk vorgesehen. Zu diesem Zweck kann die Biegezange einen Verlängerungssteg mit zwei gegenüberliegenden Enden umfassen. An einem ersten Ende des Verlängerungsstegs ist zweckmäßigerweise der erste Biegestempel angeordnet, während ein zweites Ende des Verlängerungsstegs gelenkig mit dem ersten und dem zweiten Hebel gekoppelt sein kann. Der erste Biegestempel kann einstückig mit dem Verlängerungssteg ausgebildet sein.

Die beiden ersten Gegenlager können relativ zueinander ortsfest fixiert sein oder einen je nach Betätigungszustand der Biegezange variablen Abstand zueinander aufweisen. Es kann daran gedacht werden, beide Gegenlager an ein und derselben Branche vorzusehen. Es wäre aber auch denkbar, an jeder der beiden Branchen je eines der beiden ersten Gegenlager auszubilden.

Die ersten Gegenlager oder der erste Biegestempel oder sämtliche dieser Komponenten können konturiert sein, um mit einer komplementären Kontur des chirurgischen Elements zusammenzuwirken. Eine solche Maßnahme wirkt sich bezüglich des chirurgischen Elements lagestabilisierend aus und erleichtert daher den Biegevorgang.

Gemäß einer ersten Ausführungsform ist für jede der beiden schwenkbaren Branchen eine separate Drehachse vorgesehen. Die Drehachsen der beiden Branchen sind in diesem Fall voneinander beabstandet. Gemäß einer zweiten Ausführungsform sind beide Branchen um eine gemeinsame Drehachse schwenkbar.

Bei einer multifunktionalen Ausgestaltung der Biegezangen weisen die Branchen mit gemeinsamer Drehachse je ein zweites Gegenlager für das chirurgische Element auf. Im Bereich der gemeinsamen Drehachse der Branchen ist ein zweiter, bei einer Betätigungsbewegung der Branchen ortsfester Biegestempel ausgebildet, der zum Zusammenwirken mit dem chirurgischen Element in einem Bereich zwischen den beiden zweiten Gegenlagern vorgesehen ist.

Bei einer Weiterbildung dieser Biegezange ist ein Nominalabstand zwischen dem zweiten Biegestempel und den zweiten Gegenlagern einstellbar. Zum Einstellen des Nominalabstands kann der zweite Biegestempel exzentrisch drehbar sein und/oder unterschiedliche Konturabschnitte aufweisen.

Die ersten und zweiten Gegenlager und der erste und zweite Biegestempel können an unterschiedlichen Positionen ein und derselben Biegezange vorgesehen werden. Zur Vereinfachung der Handhabung sind die ersten Gegenlager und der erste Biegestempel zweckmäßigerweise auf einer ersten Seite der Branchen ausgebildet und die zweiten Gegenlager und der zweite Biegestempel auf einer der ersten Seite gegenüberliegenden zweiten Seite der Branchen.

Gemäß einem weiteren erfindungsgemäßen Aspekt wird ein Biegezangensystem für chirurgische Elemente wie Knochenplatten bereitgestellt. Das Biegezangensystem umfasst zusätzlich zu der Biegezange das zu biegende chirurgische Element.

Das chirurgische Element, beispielsweise eine lineare Knochenplatte, kann eine Vorzugsebene aufweisen. Demgemäss kann die Biegezange ausgebildet sein, um das chirurgische Element sowohl in der Vorzugsebene als auch aus der Vorzugsebene heraus zu biegen. Das Biegen in der Vorzugsebene erfolgt zweckmäßigerweise mittels der ersten Gegenlager und dem ersten Biegestempel, während zum Biegen aus der Vorzugsebene heraus die zweiten Gegenlager und der zweite Biegestempel zum Einsatz gelangen können. Vorteilhafterweise sind im Bereich der Gegenlager und Biegestempel geeignete Anlageflächen für das zu biegende chirurgische Element ausgebildet, um dessen Vorzugsebene bezüglich der Gegenlager und Biegestempel den Erfordernissen entsprechend positionieren zu können.

### Beschreibung der Zeichnungen

Weitere Einzelheiten und Vorteile der Erfindung ergeben sich aus der nachfolgenden Beschreibung eines Ausführungsbeispiels sowie aus den Figuren. Es zeigt:
- Fig. 1: eine Aufsicht auf ein Ausführungsbeispiel einer erfindungsgemäßen Biegezange;
- Fig. 2: eine Rückansicht der Biegezange gemäß Fig. 1; und
- Fig. 3: eine Schnittansicht entlang der Linie A-A in Fig. 1.

### Beschreibung eines bevorzugten Ausführungsbeispiels

Im Folgenden wird die Erfindung anhand eines Ausführungsbeispiels einer multifunktionalen chirurgischen Biegezange für Knochenplatten erläutert. Selbstverständlich ist die Verwendung der Biegezange zum Biegen von Knochenplatten nur beispielhaft zu verstehen, da die Biegezange auch zum Biegen anderer chirurgischer Elemente wie Stäbe oder Drähte geeignet ist, die keine Vorzugsebene besitzen. Ferner ist darauf hinzuweisen, dass das Ausführungsbeispiel eine multifunktionale Biegezange mit zwei getrennten Mechanismen zum Gegenstand hat, nämlich einerseits für das Biegen der Knochenplatte in einer Vorzugsebene und andererseits zum Biegen aus der Vorzugsebene heraus. Es versteht sich von selbst, dass einer der beiden Mechanismen auch weggelassen oder abgeändert werden könnte.

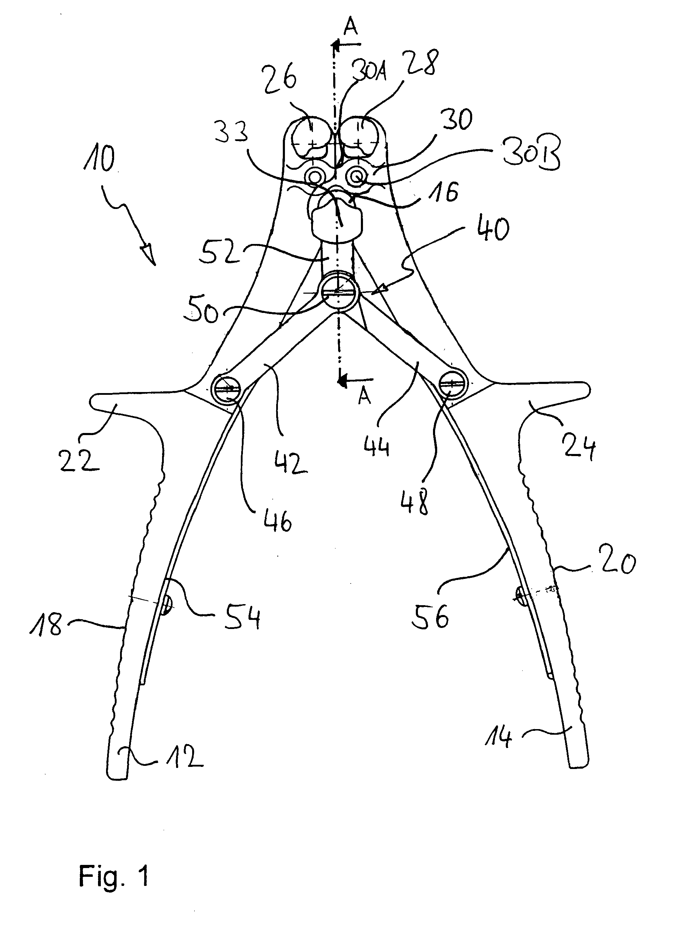

Fig. 1 zeigt eine Vorderansicht einer allgemein mit dem Bezugszeichen 10 bezeichneten Biegezange gemäß einem bevorzugten Ausführungsbeispiel mit zwei Branchen 12, 14. Die beiden Branchen 12, 14 sind im Ausführungsbeispiel um eine gemeinsame Drehachse, die in Fig. 1 durch ein Drehlager 16 definiert ist, schwenkbar. Die in Fig. 1 unteren Enden der beiden Branchen 12, 14 sind als Handgriffe 18, 20 ausgebildet. Die Handgriffe 18, 20 weisen jeweils eine strukturierte Oberfläche auf und münden in Richtung auf das Drehlager 16 in jeweils einen sich nach außen erstreckenden Vorsprung 22, 24. Die beiden Vorsprünge 22, 24 erleichtern die Bedienung der Biegezange 10 und verhindern genauso wie die strukturierten Oberflächen ein Abrutschen der Hand eines Chirurgen in Richtung auf die zu biegende Knochenplatte. Die Gefahr eines Abrutschens bestünde insbesondere dann, wenn bei dickeren Knochenplatten höhere Betätigungskräfte erforderlich werden.

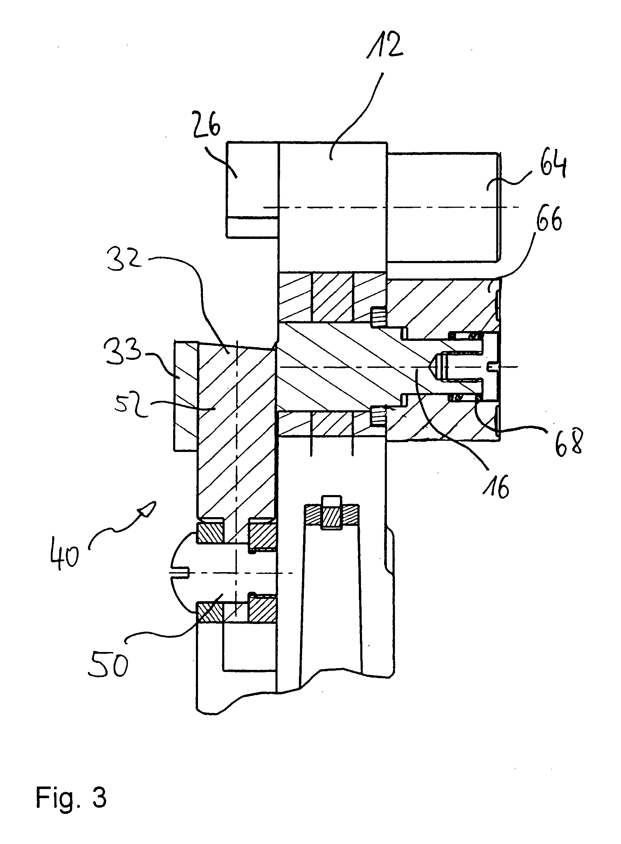

Jede der beiden Branchen 12, 14 mündet an ihrem dem jeweiligen Handgriff 18, 20 abgewandten Ende in ein rollenartiges Gegenlager 26, 28. Im Ausführungsbeispiel sind die Gegenlager 26, 28 starr mit den Branchen 12, 14 (und den Handgriffen 18, 20) gekoppelt. Die Gegenlager 26, 28 erstrecken sich in Fig. 1 aus der Zeichenebene heraus (vgl. Fig. 3) und besitzen jeweils eine grob tropfenförmige Außenkontur. Der Abstand der beiden Gegenlager 26, 28 von der gemeinsamen Drehachse der beiden Branchen 12, 14 ist deutlich kürzer als der Abstand der beiden Handgriffe 20, 22 von der Drehachse. Die daraus resultierenden Hebelverhältnisse reduzieren den zum Biegen der Knochenplatte 30 erforderlichen Kraftaufwand.

Die Außenkontur der Gegenlager 26, 28 ist an die Kontur der zu biegenden Knochenplatte (in Fig. 1 nur schematisch dargestellt und mit dem Bezugszeichen 30 bezeichnet) angepasst und im Wesentlichen hierzu komplementär. Die Konturierung wirkt sich bei einem Biegevorgang lagestabilisierend für die Knochenplatte 30 aus und vereinfacht daher die Handhabung der Biegezange 10.

In einem Bereich zwischen den beiden Gegenlagern 26, 28 (und in den Fign. 1 und 3 unterhalb dieser Gegenlager 26, 28) ist ein Biegestempel 32 vorgesehen. In Fig. 1 ist der Biegestempel 32 durch eine Linearführung 33 für den Biegestempel 32 verdeckt. Die Linearführung 33 ist in der Aufsicht gemäß Fig. 1 komplementär zur Knochenplatte 30 konturiert. Diese Konturierung erleichtert die Aufnahme der zu biegenden Knochenplatte 30.

Der Biegestempel 32 weist wie die Gegenlager 26, 28 eine ungefähr tropfenförmige Außenkontur mit einer in Richtung auf die Knochenplatte 30 weisenden Ausbuchtung (in den Figuren nicht erkennbar) auf. Er ist damit ebenfalls im Wesentlichen komplementär zur Knochenplatte 30 konturiert und dazu ausgebildet, um mit der Knochenplatte 30 in einem verschlanktem Bereich 30A (der zwischen zwei Durchgangsöffnungen 30B für Befestigungselemente wie Knochenschrauben ausgebildet ist) zusammenzuwirken.

Für den Biegestempel 32 ist eine Betätigungseinrichtung 40 vorgesehen. Die Betätigungseinrichtung 40 setzt eine Betätigungsbewegung der beiden Branchen 12, 14 in eine Linearbewegung des Biegestempels 32 in Richtung auf die beiden Gegenlager 26, 28 und damit auch in Richtung auf die Knochenplatte 30 um.

Die Betätigungseinrichtung 40 ist im Ausführungsbeispiel nach Art eines Ellipsenlenker-Getriebes ausgebildet, wobei jedoch im Gegensatz zu "konventionellen" Ellipsenlenker-Getrieben (vgl. das obengenannte Lehrbuch von S. Hildebrand) im Ausführungsbeispiel kein ortsfester Verankerungspunkt vorgesehen ist. Das Ellipsenlenker-Getriebe ist vielmehr im vorliegenden Fall mit jeder der beiden beweglichen Branchen 12, 14 und dem anzutreibenden Biegestempel 32 gekoppelt.

Im Ausführungsbeispiel umfasst die als Ellipsenlenker-Getriebe ausgebildete Betätigungseinrichtung 40 zwei gleich lange Hebel 42, 44. Der eine Hebel 42 ist über ein Gelenk 46 mit der einen Branche 12 gekoppelt und der andere Hebel 44 über ein weiteres Gelenk 48 mit der anderen Branche 14. An ihren den Branchen 12, 14 abgewandten Enden sind die beiden Hebel 42, 44 mittels eines gemeinsamen Gelenks 50 miteinander sowie mit dem Biegestempel 32 gekoppelt. Genauer gesagt sind die beiden Hebel 42, 44 am gemeinsamen Gelenk 50 mit einem den Biegestempel 32 tragenden Verlängerungssteg 52 verbunden. Im vorliegenden Fall ist der Biegestempel 32 einstückig mit dem Verlängerungssteg 52 ausgebildet. Bei einer Betätigungsbewegung der beiden Branchen 12, 14 gleitet der Verlängerungssteg 52 entlang der Linearführung 33, so dass sich die Linearführung 32 stabilisierend auf die Bewegung des Verlängerungsstegs 52 und damit auch stabilisierend auf die Bewegung des Biegestempels 32 auswirkt.

Fig. 1 zeigt die Ausgangs- oder Normalstellung der Biegezange 10. In dieser Stellung werden die beiden Handgriffe 18, 20 von in Fig. 1 nur teilweise dargestellten Blattfedern 54, 56 voneinander beabstandet gehalten. Bei einer Betätigung der Biegezange 10 ist folglich die Vorspannung der Blattfedern 54, 56 zu überwinden.

Die in Fig. 1 dargestellte Vorderseite der Biegezange 10 mit dem Biegestempel 32 und den beiden Gegenlagern 26, 28 ist dazu vorgesehen, die Knochenplatte 30 in deren Vorzugsebene zu biegen. Zu diesem Zweck wird in einem ersten Schritt die Knochenplatte 30 wie in Fig. 1 angedeutet auf die vordere Oberfläche der Biegezange 10 flächig angelegt, und zwar in einem Zwischenraum zwischen dem Biegestempel 32 einerseits und den beiden Gegenlagern 26, 28 andererseits.

Beim Anlegen der Knochenplatte 30 wird diese vom Chirurgen derart positioniert, dass der verschlankte Bereich 30A mittig bezüglich der Ausbuchtung des Biegestempels 32 platziert ist. Dabei werden automatisch die bauchigen Abschnitte der Knochenplatte 30 korrekt bezüglich der konturierten Gegenlager 26, 28 positioniert. Anschließend erfolgt bei einer Betätigung der Branchen 12, 14 unter Überwindung der Vorspannung der Blattfedern 54, 56 eine Bewegung der Branchen 12, 14 aufeinander zu.

Die Betätigungsbewegung der Branchen 12, 14 wird von der Betätigungseinrichtung 40 in eine lineare Bewegung des Biegestempels 32 in Richtung auf die Knochenplatte 30 umgesetzt. Dieses Umsetzen ist darauf zurückzuführen, dass sich die beiden Hebel 42, 44 einander annähern. Als Folge dieser Annäherungsbewegung wird das gemeinsame Gelenk 50 der beiden Hebel 42, 44 und damit auch der mit diesem Gelenk 50 gekoppelte Verlängerungssteg 52 für den Biegestempel 32 in Richtung auf die Knochenplatte 30 bewegt. Die resultierende Bewegung des Biegestempels 32 erfolgt entlang der durch die Pfeile A gekennzeichneten gestrichpunkteten Gerade.

Vorteilhaft bei der Betätigungseinrichtung 40 gemäß dem Ausführungsbeispiel ist die Tatsache, dass der Biegestempel 32 entlang einer zur Knochenplatte 30 im Wesentlichen senkrechten Geraden und nicht auf einer Ellipsenbahn geführt wird. Dies gestattet eine erhöhte Präzision beim Biegen, da keine seitlich gerichteten (Scher-) Kräfte auftreten. Vorteilhaft ist ferner, dass eine vergleichsweise geringe Betätigungsbewegung der Branchen 12, 14 in einen relativ großen linearen Versatz des Biegestempels 32 umgesetzt werden kann. Dies hat zur Folge, dass der Nominalabstand zwischen den beiden Gegenlagern 26, 28 und dem Biegestempel 32 vergleichsweise groß gehalten werden kann. Demgemäss können unterschiedlich breite Knochenplatten 30 zwischen dem Biegestempel 32 und den Gegenlagern 26, 28 positioniert und gebogen werden.

Vorteilhaft ist ferner, dass der Biegevorgang durch die Bewegung der Gegenlager 26, 28 (auf jeweils einer elliptischen Bahn) unterstützt wird. Diese Unterstützung besteht im Wesentlichen darin, dass die Knochenplatte 30 aufgrund der Bewegung der Gegenlager 26, 28 sozusagen um den Biegestempel 32 herum gebogen wird.

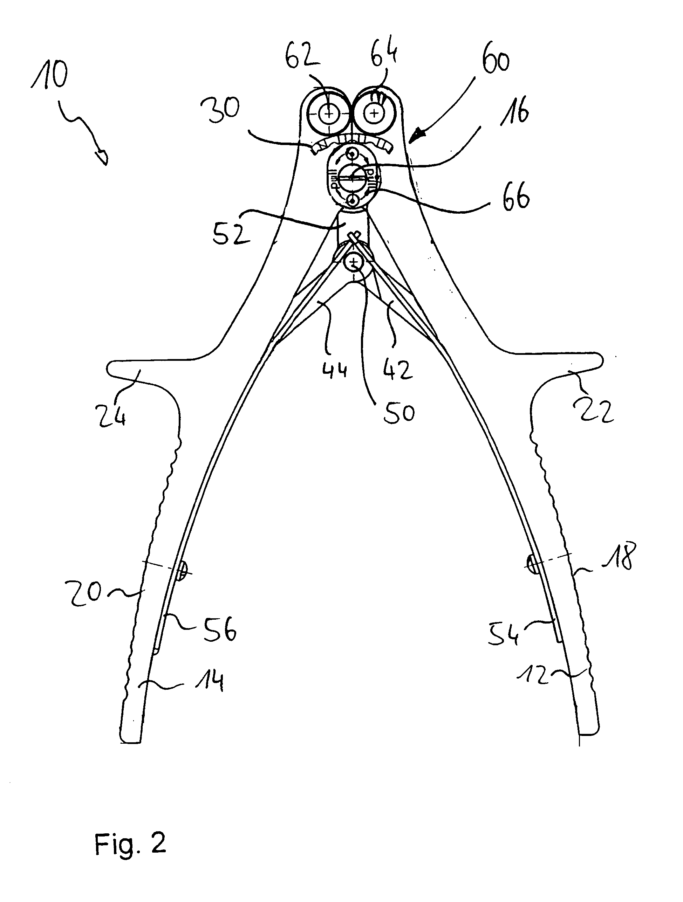

Fig. 2 zeigt eine Aufsicht auf die Rückseite der Biegezange 10 mit einer Einrichtung 60 zum Biegen der lediglich schematisch dargestellten Knochenplatte 30 aus deren Vorzugsebene heraus. Die Einrichtung 60 umfasst zwei rollenartige Gegenlager 62, 64 sowie einen Biegestempel 66. Jedes der beiden Gegenlager 62, 64 ist an einem dem jeweiligen Handgriff 18, 20 abgewandten Ende der Branchen 12, 14 ausgebildet. Die Gegenlager 62, 64 erstrecken sich in Fig. 2 aus der Zeichenebene heraus (vgl. Fig. 3) und besitzen jeweils eine kreisrunde Außenkontur. Der Abstand der beiden Gegenlager 62, 64 von der gemeinsamen Drehachse der beiden Branchen 12, 14 ist deutlich kürzer als der Abstand der beiden Handgriffe 20, 22 von der Drehachse. Die daraus resultierenden Hebelverhältnisse reduzieren den Kraftaufwand zum Biegen der Knochenplatte 30.

Der Biegestempel 66 ist bezüglich des gemeinsamen Drehlagers 16 der beiden Branchen 12, 14 drehbar. Wie Fig. 2 entnommen werden kann, besitzt der Biegestempel 66 eine elliptische Grundform und ist exzentrisch bezüglich der Drehachse 16 gelagert. Die exzentrische Lagerung des Biegestempels 66 ergibt sich auch aus der Schnittansicht gemäß Fig. 3.

Der Biegestempel 66 kann bezüglich des Drehlagers 16 in zwei unterschiedlichen Raststellungen positioniert werden. In der in den Fign. 2 und 3 gezeigten ersten Raststellung ist ein erster Abstand zwischen dem der Knochenplatte 30 zugewandten Ende des Biegestempels 66 und den der Knochenplatte 30 zugewandten Enden der Gegenlager 62, 64 eingestellt. Der Biegestempel 66 kann aus der in den Fign. 2 und 3 dargestellten Raststellung in eine um 180° bezüglich der Drehachse der Branchen 12, 14 gedrehte zweite Raststellung gebracht werden. In dieser zweiten Raststellung ist aufgrund der exzentrischen Lagerung des Biegestempels 66 ein zweiter Abstand zwischen dem Biegestempel 66 und den beiden Gegenlagern 62, 64 eingestellt, der größer als der oben genannte erste Abstand ist. Die zweite Raststellung ist daher vor allem zum Biegen dickerer Knochenplatten geeignet.

Um den Biegestempel 66 aus der ersten in die zweite Raststellung (und umgekehrt) zu bewegen, ist es in einem ersten Schritt erforderlich, den Biegestempel 66 unter Überwindung der Vorspannung einer Schraubenfeder 68 (vgl. Fig. 3) aus der Zeichenebene gemäß Fig. 2 herauszuziehen. Der herausgezogene Biegestempel 66 wird dann in einem zweiten Schritt um 180° gedreht. Nach dem Drehen des Biegestempels 66 wird dieser in einem dritten Schritt wieder losgelassen, woraufhin die Schraubenfeder 68 den Biegestempel 66 in die zweite Rastposition drängt.

Fig. 2 zeigt wie Fig. 1 den Ausgangszustand der Biegezange 10. Zum Biegen der Knochenplatte 30 wird diese zunächst wie in Fig. 2 angedeutet zwischen einerseits den Gegenlagern 62, 64 und andererseits dem Biegestempel 66 derart positioniert, dass die Stirnseite der flächigen Knochenplatte 30 an der Oberfläche der Biegezange 10 (genauer gesagt an den Oberflächen der Branchen 12, 14) anliegt. Die Knochenplatte 30 wird also hochkant positioniert. Da der Abstand zwischen den Gegenlagern 62, 64 und dem Biegestempel 66 in der in Fig. 1 dargestellten Ausgangsstellung nur geringfügig größer als die Dicke der Knochenplatte 30 ist, wird die Lage der Knochenplatte 30 von den Gegenlagern 62, 64 und dem Biegestempel 66 stabilisiert.

Nach dem Positionieren der Knochenplatte betätigt der Chirurg die Branchen 12, 14 unter Überwindung der Vorspannung der Blattfedern 54, 56 aufeinander zu. Bei dieser Betätigungsbewegung der Branchen 12, 14 bleibt der Biegestempel 66 ortsfest. Die Gegenlager 62, 64 bewegen sich hingegen auf elliptischen Bahnen um den Biegestempel 66 herum. Von dieser Bewegung der Gegenlager 62, 64 wird die zwischen den Gegenlagern 62, 64 und dem Biegestempel 66 positionierte Knochenplatte 30 erfasst und somit um den Biegestempel 66 herum gebogen. Da die Vorzugsebene der Knochenplatte 30 senkrecht zur Drehachse der beiden Branchen 12, 14 verläuft, wird die Knochenplatte 30 aus ihrer Vorzugsebene heraus gebogen.

Die Erfindung wurde anhand eines bevorzugten Ausführungsbeispiels erläutert. Es sind jedoch zahlreiche Änderungen und Modifikationen denkbar. Die Erfindung kann daher, innerhalb des Umfangs der nachfolgenden Ansprüche, auch abweichend von der obigen Darstellung ausgeführt werden.

## Patentansprüche

1. Biegezange (10) für chirurgische Elemente wie Knochenplatten (30), mit
- zwei relativ zueinander schwenkbaren Branchen (12, 14), die mit zusammen zwei ersten Gegenlagern (26, 28) für das zu biegende chirurgische Element gekoppelt sind;
- einem ersten Biegestempel (32) zum Zusammenwirken mit dem chirurgischen Element in einem Bereich zwischen den beiden ersten Gegenlagern (26, 28); und
- einer Betätigungseinrichtung (40) für den ersten Biegestempel (32), welche eine Betätigungsbewegung der Branchen (12, 14) in eine Linearbewegung des ersten Biegestempels (32) in Richtung auf das chirurgische Element umsetzt, **gekennzeichnet dadurch, dass** sich die ersten Gegenlager (26, 28) während der Betätigungsbewegung der Branchen (12, 14) auf jeweils einer elliptischen Bahn auseinander bewegen.

2. Biegezange nach Anspruch 1, **dadurch gekennzeichnet,**
**dass** die Betätigungseinrichtung als Getriebe (40) ausgebildet ist.

3. Biegezange nach Anspruch 2, **dadurch gekennzeichnet,**
**dass** das Getriebe (40) mit jeder der Branchen (12, 14) und mit dem Biegestempel (32) gelenkig (46, 48, 50) gekoppelt ist.

4. Biegezange nach einem der Ansprüche 1 bis 3, **dadurch gekennzeichnet,**
**dass** die Betätigungseinrichtung (40) wenigstens einen ersten Hebel (42) umfasst, der mit einer ersten der Branchen (12) und mit dem ersten Biegestempel (32) gelenkig (46, 50) gekoppelt ist.

5. Biegezange nach Anspruch 4, **dadurch gekennzeichnet,**
**dass** die Betätigungseinrichtung einen zweiten Hebel (44) umfasst, der mit einer zweiten der Branchen (14) und mit dem ersten Biegestempel (32) gelenkig (48, 50) gekoppelt ist.

6. Biegezange nach Anspruch 5, **dadurch gekennzeichnet,**
**dass** der erste und der zweite Hebel (42, 44) an einem gemeinsamen Gelenk (50) miteinander und mit dem ersten Biegestempel (32) gekoppelt sind.

7. Biegezange nach Anspruch 5 oder 6, **dadurch gekennzeichnet,**
**dass** die Biegezange (10) einen Verlängerungssteg (52) mit zwei gegenüberliegenden Enden umfasst, wobei der erste Biegestempel (32) an einem ersten der Enden angeordnet ist und ein zweites der Enden gelenkig (50) mit dem ersten und dem zweiten Hebel (42, 44) gekoppelt ist.

8. Biegezange nach einem der Ansprüche 1 bis 7, **dadurch gekennzeichnet,**
**dass** eine Linearführung (33) zur Stabilisierung der Linearbewegung des ersten Biegestempels 32 vorgesehen ist.

9. Biegezange nach einem der Ansprüche 1 bis 8, **dadurch gekennzeichnet,**
**dass** die ersten Gegenlager (26, 28) und/oder der erste Biegestempel (32) konturiert sind/ist, um mit einer komplementären Kontur des chirurgischen Elements zusammenzuwirken.

10. Biegezange nach einem der Ansprüche 1 bis 9, **dadurch gekennzeichnet,**
**dass** die beiden Branchen (12, 14) um eine gemeinsame Drehachse (16) schwenkbar sind.

11. Biegezange nach Anspruch 10, **dadurch gekennzeichnet,**
**dass** die Branchen (12, 14) je ein zweites Gegenlager (62, 64) für das chirurgische Element aufweisen und im Bereich der gemeinsamen Drehachse (16) der Branchen (12, 14) ein zweiter, bei einer Betätigungsbewegung der Branchen (12, 14) ortsfester Biegestempel (66) ausgebildet ist, der zum Zusammenwirken mit dem chirurgischen Element in einem Bereich zwischen den beiden zweiten Gegenlagern (62, 64) vorgesehen ist.

12. Biegezange nach Anspruch 11, **dadurch gekennzeichnet,**
**dass** ein Nominalabstand zwischen dem zweiten Biegestempel (66) und den zweiten Gegenlagern (62, 64) einstellbar ist.

13. Biegezange nach einem der Ansprüche 10 bis 12, **dadurch gekennzeichnet,**
**dass** die ersten Gegenlager (26, 28) und der erste Biegestempel (32) auf einer ersten Seite der Branchen (12, 14) ausgebildet sind und die zweiten Gegenlager (62, 64) und der zweite Biegestempel (66) auf einer der ersten Seite gegenüberliegendenden zweiten Seite der Branchen (12, 14) ausgebildet sind.

14. Biegezangensystem für chirurgische Elemente wie Knochenplatten (30), umfassend die Biegezange (10) nach einem der Ansprüche 1 bis 13 und das zu biegende chirurgische Element.

15. Biegezangensystem nach Anspruch 14, **dadurch gekennzeichnet,**
**dass** das chirurgische Element eine Vorzugsebene aufweist und die Biegezange (10) ausgebildet ist, um das chirurgische Element sowohl in der Vorzugsebene als auch aus der Vorzugsebene heraus zu biegen.

16. Biegezangensystem nach Anspruch 15, **dadurch gekennzeichnet,**
**dass** die ersten Gegenlager (26, 28) und der erste Biegestempel (32) zum Biegen in der Vorzugsebene und die zweiten Gegenlager (62 ,64) und der zweite Biegestempel (66) zum Biegen aus der Vorzugsebene heraus ausgebildet sind.

## Claims

1. Bending forceps (10) for surgical elements such as bone plates (30), comprising
- two branches (12, 14) which are pivotable relative to one another and which are coupled with two first counter bearings (26, 28) for the surgical element to be bent;
- a first bending punch (32) for co-operation with the surgical element in a region between the two first counter bearings (26, 28); and
- an actuating device (40) for the first bending punch (32), which converts an actuating movement of the branches (12, 14) into a linear movement of the first bending punch (32) in a direction towards the surgical element, **characterised in that** the first counter bearings (26, 28) each move apart from each other along an elliptical path during the actuating movement of the branches (12, 14).

2. The bending forceps according to claim 1, **characterised in that** the actuating device is designed as a gear mechanism (40).

3. The bending forceps according to claim 2, **characterised in that** the gear mechanism (40) is coupled in an articulated manner (46, 48, 50) to each of the branches (12, 14) and to the bending punch (32).

4. The bending forceps according to one of the claims 1 to 3, **characterised in that** the actuating device (40) comprises at least a first lever (32), which is coupled in an articulated manner (46, 50) to a first of the branches (12) and to the first bending punch (32).

5. The bending forceps according to claim 4, **characterised in that** the actuating device comprises a second lever (44) which is coupled in an articulated manner (48, 50) to a second of the branches (14) and to the first bending punch (32).

6. The bending forceps according to claim 5, **characterised in that** the first and the second levers (42, 44) are coupled at a common joint (50) to one another and to the first bending punch (32).

7. The bending forceps according to claim 5 or 6, **characterised in that** the bending forceps (10) comprises and extension arm (52) witch two oppositely lying ends, wherein the first bending punch (32) is arranged on a first of the ends and a second of the ends is coupled in an articulated manner (50) to the first and second levers (42,44).

8. The bending forceps according to one of the claims 1 to 7, **characterised in that** a linear guide (33) is provided for stabilizing the linear movement of the first bending punch (32).

9. The bending forceps according to one of the claims 1 to 8, **characterised in that** the first counter bearings (26, 28) and/or the first bending punch (32) are/is contoured in order to co-operate with a complementary contour of the surgical element.

10. The bending forceps according to one of the claims 1 to 9, **characterised in that** the two branches (12, 14) are pivotable about a common axis of rotation (16).

11. The bending forceps according to claim 10, **characterised in that** the branches (12, 14) each comprise a second counter bearing (62, 64) for the surgical element, and in the region of the common axis of rotation (16) of the branches (12, 14) a second bending punch (66) is formed which is stationary during an actuation movement of the branches (12, 14), which is provided for co-operation with the surgical element in a region between the two second counter bearings (62, 64).

12. The bending forceps according to claim 11, **characterised in that** the nominal distance between the second bending punch (66) and the second counter bearings (62, 64) is adjustable.

13. The bending forceps according to one of the claims 10 to 12, **characterised in that** the first counter bearings (26, 28) and the first bending punch (32) are formed on a first side of the branches (12, 14) and the second counter bearings (62, 64) and the second bending punch (66) are formed on a second side of the branches (12, 14) lying opposite to the first side.

14. A bending forceps system for surgical elements such as bone plates (30), comprising a bending forceps (10) according to one of the claims 1 to 13 and the surgical element to be bent.

15. A bending forceps system according to claim 14, **characterised in that** the surgical element has a preferred plane and the bending forceps (10) is designed so as to bend the surgical element in the preferred plane and also outwardly from the preferred plane.

16. A bending forceps system according to claim 15, **characterised in that** the first counter bearings (26, 28) and the first bending punch (32) are designed for bending in the preferred plane, and the second counter bearings (62, 64) and the second bending punch (66) are designed for bending outwardly from the preferred plane.

## Revendications

1. Pince à cintrer (10) pour des éléments chirurgicaux tels que des plaques d'ostéosynthèse (30), comprenant
- deux branches (12, 14) pouvant pivoter l'une par rapport à l'autre, qui sont couplées par deux premières butées (26, 28) pour l'élément chirurgical à cintrer ;
- un premier poinçon de cintrage (32) destiné à coopérer avec l'élément chirurgical dans une zone comprise entre les deux premières butées (26, 28) ; et
- un dispositif d'actionnement (40) pour le premier poinçon de cintrage (32), lequel transforme un mouvement d'actionnement des branches (12, 14) en un mouvement linéaire du premier poinçon de cintrage (32) en direction de l'élément chirurgical,
**caractérisée en ce que** les premières butées (26, 28) s'écartent l'une de l'autre en suivant chacune une trajectoire elliptique pendant le mouvement d'actionnement des branches (12, 14).

2. Pince à cintrer selon la revendication 1,
**caractérisée en ce que** le dispositif d'actionnement est réalisé sous la forme d'un mécanisme de transmission (40).

3. Pince à cintrer selon la revendication 2,
**caractérisée en ce que** le mécanisme de transmission (40) est couplé de manière articulée (46, 48, 50) à chacune des branches (12, 14) et au poinçon de cintrage (32).

4. Pince à cintrer selon une des revendications 1 à 3,
**caractérisée en ce que** le dispositif d'actionnement (40) comprend au moins un premier levier (42) qui est couplé de manière articulée (46, 50) à une première (12) des branches et au premier poinçon de cintrage (32).

5. Pince à cintrer selon la revendication 4,
**caractérisée en ce que** le dispositif d'actionnement comprend un deuxième levier (44) qui est couplé de manière articulée (48, 50) à une deuxième (14) des branches et au premier poinçon de cintrage (32).

6. Pince à cintrer selon la revendication 5,
**caractérisée en ce que** les premier et deuxième leviers (42, 44) sont couplés entre eux et au premier poinçon de cintrage (32) à une articulation commune (50).

7. Pince à cintrer selon la revendication 5 ou 6,
**caractérisée en ce que** la pince à cintrer (10) comprend un bras prolongateur (52) avec deux extrémités opposées, le premier poinçon de cintrage (32) étant disposé à une première des extrémités et une deuxième des extrémités étant couplée de manière articulée (50) aux premier et deuxième leviers (42, 44).

8. Pince à cintrer selon une des revendications 1 à 7,
**caractérisée en ce qu'**un guidage linéaire (33) est prévu pour stabiliser le mouvement linéaire du premier poinçon de cintrage (32).

9. Pince à cintrer selon une des revendications 1 à 8,
**caractérisée en ce que** les premières butées (26, 28) et/ou le premier poinçon de cintrage (32) présente(nt) un contour pour coopérer avec un contour complémentaire de l'élément chirurgical.

10. Pince à cintrer selon une des revendications 1 à 9,
**caractérisée en ce que** les deux branches (12, 14) peuvent pivoter autour d'un axe de rotation commun (16).

11. Pince à cintrer selon la revendication 10,
**caractérisée en ce que** les branches (12, 14) comportent chacune une deuxième butée (62, 64) pour l'élément chirurgical et un deuxième poinçon de cintrage (66), fixe lors d'un mouvement d'actionnement des branches (12, 14), est formé dans la zone de l'axe de rotation commun (16) des branches (12, 14), lequel est prévu pour coopérer avec l'élément chirurgical dans une zone comprise entre les deux deuxièmes butées (62, 64).

12. Pince à cintrer selon la revendication 11,
**caractérisée en ce qu'**une distance nominale entre le deuxième poinçon de cintrage (66) et les deuxièmes butées (62, 64) est réglable.

13. Pince à cintrer selon une des revendications 10 à 12,
**caractérisée en ce que** les premières butées (26, 28) et le premier poinçon de cintrage (32) sont formées d'un premier côté des branches (12, 14) et les deuxièmes butées (62, 64) et le deuxième poinçon de cintrage (66) d'un deuxième côté des branches (12, 14) opposé au premier côté.

14. Système de pince à cintrer pour des éléments chirurgicaux tels que des plaques d'ostéosynthèse (30), comprenant la pince à cintrer (10) selon une des revendications 1 à 13 et l'élément chirurgical à cintrer.

15. Système de pince à cintrer selon la revendication 14,
**caractérisé en ce que** l'élément chirurgical présente un plan préférentiel et la pince à cintrer (10) est formée de manière à cintrer l'élément chirurgical aussi bien dans le plan préférentiel que hors du plan préférentiel.

16. Système de pince à cintrer selon la revendication 15,
**caractérisé en ce que** les premières butées (26, 28) et le premier poinçon de cintrage (32) sont conçues pour le cintrage dans le plan préférentiel et les deuxièmes butées (62 ,64) et le deuxième poinçon de cintrage (66) pour le cintrage hors du plan préférentiel.
